# EUROPEAN PATENT APPLICATION

(11) **EP 3 539 448 A2**
(43) Date of publication of application: **18.09.2019**
(21) Application number: 19156793.2
(22) Date of filing: 04.02.2013
(51) Int. Cl.: A61B 1/00, A61B 1/227, A61B 1/05

(54) **METHOD FOR IDENTIFYING OBJECTS IN A SUBJECT'S EAR**

(62) Divisional of application: 13000553.1
(71) Applicant: Helen of Troy, Limited, St. Michael (BB)
(72) Inventor: RUPPERSBERG, J. Peter, 1807 Blonay (CH); LEPPLE-WIENHUES, Albrecht, 25300 Pontarlier (FR)
(74) Representative: Ruttensperger Lachnit Trossin Gomoll

(57) **Abstract**

The present invention refers to a method of identifying objects in a subject's ear, comprising the following steps: introducing at least one electronic imaging unit and at least one light source into an ear canal of a subject's outer ear; using the at least one electronic imaging unit to capture at least one image; and determining brightness and/or color information to identify objects shown in the at least one image.

## Description

The invention refers to a method of identifying objects in a subject's ear. Looking into ears is called "otoscopy". Otoscopy is a standard medical examination technique established more than 100 years ago. Medical students learn otoscopy early in their studies during the practical course in physiology. Otoscopic examination assists the skilled physician in examining the ear canal or eardrum which may be affected e.g. by otitis media, middle ear hydrops, otitis externa, and eardrum perforation. Object recognition in otoscopy is also directed to the identification of particles or any material, e.g. hair, earwax, foreign objects, etc., which may obstruct the ear canal or coat the eardrum. Such applications are highly desired for routine care.

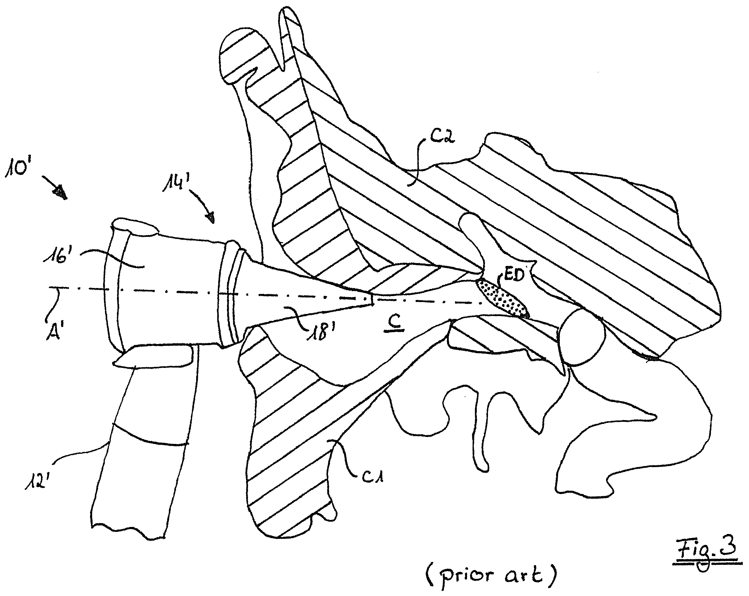

To perform otoscopy, a medical device called "otoscope" (sometimes also "auriscope") is used. A typical otoscope 10' is shown in figure 3. The otoscope 10' comprises a handle portion 12' allowing the user to manipulate the otoscope during its application. The term "to manipulate" in this context refers to different kinds of manipulation, such as - but not limited to - holding the otoscope, aligning the otoscope with respect to the subject's ear, and turning on or off a light. The otoscope 10' further comprises a head portion 14' connected to the handle portion 12'. The head portion 14' exhibits a substantially tapering form - usually a conical form - extending along a longitudinal axis A' of the head portion 14'. The head portion 14' is substantially comprised of an empty funnel, wherein the tip of the funnel typically has a diameter of 3mm. Furthermore, the head portion 14' has a proximal end 16' adjacent to the handle portion 12' and a smaller distal end 18' configured to be introduced into an ear canal C of a subject's outer ear. The term "end" in this context does not mean a single point but rather refers to a region or section of the head portion 14', wherein the proximal end 16' is located opposite to the distal end 18' with respect to the longitudinal axis A'. The ear canal C is partly surrounded by soft connective tissue C1 and - further down towards the middle ear - partly by hard bone C2.

If otoscopic methods of the art are applied e.g. to examine the subject's eardrum (as an object), the 3mm tip has to be pushed deeply into the ear canal C while observing and simultaneously illuminating the subject's eardrum ED through the empty funnel. Normally, due to the natural curvature of the ear canal C, the eardrum ED is not visible from outside the ear. In order to overcome the natural curvature of the ear canal C, the skilled physician has to carefully pull the outer ear upward and to the back while carefully pushing the tip of the funnel into the ear canal as deeply as necessary to display the eardrum. The ear canal C has to be deformed in such a way that the physician has a free view onto the eardrum ED along the optical axis of the otoscope 10', wherein the optical axis corresponds to the longitudinal axis A' of the head portion 14'. The optics of an otoscope is situated only at the wider end of the funnel, i.e. at the proximal end 16' of the head portion 14', and essentially consists of a lamp and a lens (not shown) to magnify the image of the eardrum ED.

The otoscopy procedure needs manual skills and significant training to carefully push the funnel into the ear canal C while looking inside and manipulating the curvature of the ear canal C by pulling the ear. For example, the trained physician is well aware to brace the hand holding of the otoscope against the subject's head to avoid injury to the ear canal C and the eardrum ED by placing the index finger or little finger against the head. In particular in young children - where the inner part of the ear canal is relatively short and where sudden head movement during examination may often occur - risk of penetration of the sensitive ear canal skin or even of the eardrum ED exists. Other than pain and handicapped hearing, such an injury is also known to potentially induce cardiovascular complication through vagal overstimulation and, therefore, has to be avoided under all circumstances.

Furthermore, especially in an inflamed ear, the mechanical manipulation of "straightening" the ear canal C typically causes considerable discomfort or even pain, rendering the examination of an infant even more difficult.

For any application of an otoscope or its mode of use, it is desired to allow its user to distinguish the objects located in the ear canal or at its end, in particular the eardrum itself of any objects adhering to the eardrum.

For the above reasons, reliably and securely handling an otoscope of the art is currently subject to only well trained physicians and not amenable to the larger community of practitioners. A study recently published in the US as a result of a survey has shown that even physicians often fail to (correctly) determine the status of e.g. the subject's eardrum or fail to correctly interpret the image provided by the otoscope (i.e. correct and meaningful object recognition). Such failures result in misinterpretation of the status of the inner ear canal or the eardrum. As a consequence, e.g. over-medication with antibiotics for treating supposed inflammations of the eardrum occurs, because physicians tend to err on the side of caution, or meaningless image interpretation occurs.

Notably, there also exist other otoscopic devices, as e.g. video otoscope, allowing a skilled expert to capture images of the subject's eardrum and the ear canal. Such video otoscopes comprise a bundle of light guides extending from the distal end of the head portion to a CCD-chip located remote from the distal end. The achievable resolution of the images depends on the number of light guides. In order to obtain images having a satisfying resolution, a significant number of individual light guides must be provided rendering devices by far too expensive for routine care. Moreover, all of the known video otoscopes having the CCD-chip located remote from the distal end of the head portion require superior handling skills by he physician. For the above reasons, they are not configured and suitable for domestic use by a larger community of practitioners, nor use by laypersons.

The otoscopic methods known in the art are - as a matter of fact - subject to the structural and geometrical characteristics of otoscopes as described above. All otoscopes currently on the market - including video otoscopes - generally are based on the following fundamental design: a relatively thin open funnel. Length, angle, field of vision and size of the funnels are essentially similar for all marketed otoscopes. As a result of these common characteristics, ease of use (due to safety issues) is limited for such devices. Methods for reliable detection of objects in the ear canal, including the eardrum, are remarkably intricate with such known otoscopes.

Consequently, until today otoscopy has almost exclusively been applied by well-trained medical doctors. However, it would be desirable to extend the capability of otoscopy beyond the trained professionals. Due to its broad spectrum of applications, it should be made amenable to any layperson, such as parents, who may desire to e.g. examine whether dirt or particles is/are located in the children's ear canal.

It is therefore an object of the present invention to provide a method that allows for reliable identification of objects in the subject's ear and that preferably shall be also domestically applied by laypersons without any - or at least with a significantly reduced - risk of causing injuries to the subject.

That object is achieved according to the present invention by a method exhibiting the features of item 1. Preferred embodiments of the present invention are provided by the dependent items.

In particular, that object is achieved by a method of identifying objects in a subject's ear, comprising the following steps: introducing at least one electronic imaging unit and at least one light source into an ear canal of a subject's outer ear; using the at least one electronic imaging unit to capture at least one image; and determining brightness and/or color information to identify objects shown in the at least one image.

As described above, the optics of an otoscope adapted to carry out the otoscopic method according to art - comprising a lamp and a lens - is positioned exclusively at the wider end of the funnel, i.e. at the proximal end of the head portion. As a consequence, the longitudinal axis of the head portion forms the optical axis of the otoscope. The optical axis has to directly point to the eardrum for enabling visual access through the ear canal to the eardrum. In order to enable such visual access state of the art methods require the practitioner to significantly deform the subject's ear and further require introducing a relatively narrow tip of the funnel deeply into the subject's ear canal.

Introducing at least one electronic imaging unit and/or at least one light source (preferably both) into an ear canal of a subject's outer ear - according to the method of the present invention - overcomes these disadvantages of such prior art methods using known otoscopes. That is, the optical axis of an otoscope used for carrying out the method of the present invention does not have to correspond to the longitudinal axis of the head portion. Instead, the optical axis of the electronic imaging unit may be arranged at an angle with respect to the longitudinal axis, allowing the device to "look around the corner". Consequently, when carrying out the method of the present invention, the requirement to deform the subject's ear is eliminated or greatly reduced. Furthermore, the inventive method avoids the risk of injury to the ear canal or eardrum by allowing the use of otoscopes with a tip of the head portion that exhibit significantly larger dimensions as compared to an otoscope according to the art. Thus, the risk of introducing the head portion of the otoscope too deeply into the subject's ear canal is considerably reduced. Both improvements pave the way to allow laypersons to carry out the method according to the invention.

As a further advantage of the present inventive method it enables the use of imaging devices which provide a larger field of vision. The field (or angle) of vision of the electronic imaging unit of such devices is positioned at the distal end of the head portion. Thereby, a much larger the field (or angle) of vision is obtainable than by methods which are based on the relatively acute empty funnel of an otoscope according to the prior art.

Once at least one image has been captured by the at least on electronic imaging unit, object recognition and unambiguous object identification (e.g. distinguishing objects, such as earwax, hair, and the eardrum) can be performed by determining brightness and/or color information of the pixels of the at least one captured image. Each pixel of the image obtained by the electronic imaging unit is characterized by a numerical value corresponding to the brightness of that pixel and - if the electronic imaging device is a color imaging device - also by a numerical value corresponding to the color of that pixel. Different objects can be identified e.g. by their typical color.

For improved object identification the method according to the present invention preferably further comprises the following steps: using the at least one electronic imaging unit to capture at least two images from different positions within the ear canal and/or with illumination from different positions within the ear canal; and comparing the at least two captured images with each other to identify objects shown in the images.

Thus, the above object is solved by a method of identifying objects in a subject's ear may comprise the following steps: introducing at least one electronic imaging unit and at least one light source into an ear canal of a subject's outer ear; using the at least one electronic imaging unit to capture at least two images from different positions within the ear canal and/or with illumination from different positions within the ear canal; comparing the at least two captured images with each other to reliably identify objects shown in the images.

With these features, the at least one electronic imaging unit is suitable to capture at least two images from different positions within the ear canal, e.g. by relocating one single electronic imaging unit when placed in the subject's ear canal and/or by providing images from two or more electronic imaging units positioned at different sites in the ear canal. Alternatively or additionally, the method may be based on the implementation of at least one illumination unit which is adapted to illuminate objects within the ear canal from different positions (e.g. from two or more positions). Preferably, a combination of both approaches is realized by the inventive method, which allows capturing images from different positions under differing illumination conditions. Such a mode of action allows for reliable identification of distinct objects (e.g. the eardrum, particles of earwax, hair, etc. in the subject's ear), as will be described in more detail below. Thereby, the risk of image misinterpretation and failure in object recognition is significantly reduced.

If at least two images are captured from different positions within the ear canal, different objects, such as the eardrum and other objects are discriminated by comparing their positions as provided in the at least two images. That is, the inventive method makes it possible - in contrast to prior art methods - to determine the distance of various objects in the ear canal with respect to the electronic imaging unit according to the fundamental principle of stereoscopic viewing, also known as "parallax". Parallax is a displacement or difference in the apparent position of an object viewed along two different lines of sight, and is measured by the angle or semi-angle of inclination between those two lines. For example, a person closing only his left eye sees objects being relatively close at a position other than by closing only his right eye. However, the person will see relatively remote objects substantially at the same position. The human brain is thus able to determine the distance from the observer to the objects as a result of the parallax phenomenon. The same approach may be realized according to the present inventive method by the use of electronic means, such as a logic unit, when capturing images from different positions within the ear canal. Since the electronic imaging unit will not and cannot be introduced too deeply into the subject's ear canal according to the inventive method, the eardrum, as the membrane (object) terminating the ear canal, is relatively remote with respect to the electronic imaging unit, whereas other objects in the ear canal positioned more proximal to the electronic imaging unit are recognized as being less remote from imaging unit as reference point. Thus, e.g. the eardrum can be readily distinguished from other objects located more proximal in the ear canal by the inventive method.

Alternatively or additionally, different objects, such as earwax, hair, and the eardrum, within the subject's ear canal may be discriminated by comparing their appearance as depicted by at least two images captured under illumination from different positions (for each single image) within the ear canal. If an object positioned relatively closely to the electronic imaging unit, such as earwax, is illuminated from different positions within the ear canal (by e.g. two or more distinct light sources or by e.g. one single light source which can be repositioned when carrying out the inventive method), the appearance of such an object will significantly differ in the at least two images captured according to the inventive method. Usually, the position of the sources of illumination is chosen such that, when carrying out the inventive method, they are still positioned closely to the electronic imaging unit. In contrast thereto, an object positioned relatively remote from the electronic imaging unit, such as the eardrum, will typically not change its appearance in the at least two images captured according to the inventive method by such illumination from different positions.

The method according to the present invention preferably further comprises a step of generating a calculated image based on the at least two captured images. One mode of carrying out the inventive method may be directed to exclusive object recognition of the eardrum. Thereby, the calculated image preferably does not display other more proximal (located more closely to the electronic imaging unit) objects, such as earwax and hair.

Under such circumstances, any object in the ear canal, e.g. a hair, which - at least partially - obstructs the view of the electronic imaging unit at a certain position within the ear canal onto the eardrum, may not prevent the user from obtaining the desired image information. The inventive method still allows to provide either a free view onto the eardrum by the electronic imaging unit, as the method allows to relocate the imaging unit to another position in the ear canal or may thereby at least provide a free view onto the part of the eardrum that was previously partially obstructed by the hair. For such an embodiment of the invention, the objects located relatively closely to the electronic imaging unit, such as earwax and hair, will be preferably identified as well, whereby the inventive method may provide an additional step, e.g. by electronic means, such as a logic unit, of generating a calculated image. Such a calculated image would not display any objects located relatively closely to the electronic imaging unit, such as earwax and hair, if the inventive method - as described for that embodiment - were intended to capture the best image possible of the eardrum. Consequently, an image will be calculated by the inventive method exclusively depicting the eardrum (and its structure), whereas other objects, such as hair and earwax, have been "eliminated" upon their recognition.

The term "relatively closely" in this context shall refer to a distance of preferably not more than 6mm, more preferably of no more than 4mm from the reference point, e.g. the electronic image unit.

The image calculated according to the inventive method may be provided to a user by a display device, or may be stored to a storage card, or may be transferred to an external device via cable or wirelessly. If the calculated image is stored, the user, be it a lay person or a physician, may later analyze the image for whatever purpose.

If the at least one electronic imaging unit is a color video camera, the method according to the present invention preferably further comprises a step of determining the degree of reddishness of any physiological objects in the ear canal (skin of the ear canal or of the eardrum), once the desired object (e.g. the eardrum) has been identified. Determining the degree of reddishness of e.g. the eardrum may help the layperson to decide as to whether a physician should be visited or not, as it may potentially indicate inflammation of the eardrum. Inflammation of the eardrum may suggest e.g. a (bacterial/viral) infection. Any such more advanced or final disease diagnosis has to be carried out by the physician on the basis of other symptoms exhibited by the subject, which are observed by the physician, or by the physician's further examination. Disease diagnosis can therefore not be derived from the output provided by the method according to the invention, e.g. image information alone. Reddishness may also be observed elsewhere in the ear canal, as e.g. inflammation may also affect the inner part of the ear canal of the subject's outer ear. Thus, the method according to the present invention may additionally or alternatively determine the degree of reddishness of the inner part of the ear canal of the subject's outer ear, upon object recognition of the inner part of the ear canal by the inventive method.

The inventive method is based on an electronic imaging unit which preferably is a wide angle video camera. The term "wide angle" in this context refers to field of view angles of at least 80°, preferably of at least 110°, e.g. 120°. A method based on such wide angle cameras allows for detection of the subject's eardrum, even if the optical axis ("viewing direction") of the camera is not directly centered to the eardrum when applying the inventive method. The same holds if the eardrum is located - by applying the inventive method - relatively remote from the camera, compared to the distance between the eardrum and the tip end of an otoscope of the art during application. The electronic imaging unit used by a method of the invention may be a wafer-level camera of a substantially flat configuration, having dimensions of less than 3mm x 3mm, preferably less than 2mm x 2mm, even more preferable of about 1mm x 1mm or even less than 1mm x 1mm. Such a wafer-level camera can be produced nowadays extremely small in size with only about 3 microns per pixel. Therefore, wafer-level imaging technology allows for obtaining images of "sufficient" resolution of the eardrum, e.g. images of 250 pixels x 250 pixels, with a footprint of the camera including lens of only about 1mm x 1mm or even smaller.

The method according to the present invention preferably further comprises a step of informing the user correspondingly, if identification of the eardrum has failed. For example, it may be impossible for the electronic imaging unit to detect the eardrum and or the inner part of the ear canal - irrespective of the position of the electronic imaging unit within the ear canal - because the ear canal is blocked by massive earwax or other particles. Alternatively, the eardrum may not be identified because the user did not carry out the inventive method due to inappropriate handling of the corresponding device (otoscope). In such a case, the user may try to repeat to carry out the method according to the present invention by re-adapting the position of the otoscope device in a correct manner.

In the method according to the present invention, the at least one electronic imaging unit and/or the at least one light source is preferably displaced within the ear canal of the subject's outer ear along a predetermined path and/or by a predetermined distance between the moment of capturing a first image and the moment of capturing a second image. In order to allow for a relatively simple structural implementation of a corresponding motion mechanism for displacing the at least one electronic imaging unit and/or the at least one light source within the ear canal, the predetermined path has preferably a circular form. Moreover, in order to clearly see a difference between the positions of an object shown in two images captured from different positions within the ear canal (according to the parallax phenomenon described above), the predetermined distance preferably amounts to at least about 1 mm.

Preferably, the at least one electronic imaging unit and the at least one light source are introduced into the ear canal of the subject's outer ear no further than to a distance from the eardrum of at least a few millimeters, preferably of at least 3mm. This securely avoids injuries of the eardrum. As mentioned above, in order to avoid deeper introduction, the tip of the head portion of an otoscope adapted to carry out the method according to the present invention can exhibit greater dimensions compared to the otoscopes known in the art.

In order to carry out the method of the present invention, preferably an otoscope is used, comprising a handle portion allowing a user to manipulate the otoscope during its application; and a head portion exhibiting a substantially tapering form extending along a longitudinal axis of the head portion, wherein the head portion has a proximal end adjacent to the handle portion and a smaller distal end configured to be introduced into the ear canal of the subject's outer ear. These features are also known from an otoscope of the art as described above. However, the otoscope used for carrying out the present invention preferably further comprises the at least one electronic imaging unit positioned in the distal end of the head portion.

The electronic imaging unit may be positioned substantially centrically with respect to the longitudinal axis of the head portion. If the electronic imaging unit is positioned on the longitudinal axis of the head portion, the substantially flat electronic imaging unit is preferable inclined with respect of the longitudinal axis of the head portion, so that the optical axis (or the "main viewing direction") of the electronic imaging unit is angled with respect to the longitudinal axis of the head portion, allowing the otoscope to "look around the corner". Consequently, the otoscope adapted to carry out the method according to the present invention does not have to bee introduced as deeply into the subject's ear compared to a conventional otoscope of the prior art.

Alternatively, the electronic imaging unit may be positioned radially offset from the longitudinal axis of the head portion. Such a configuration also allows obtaining a free view onto the eardrum without having to introduce the electronic imaging unit as deeply as it would be necessary if the electronic imaging unit were placed just centrally on the longitudinal axis of the head portion. The offset may be at least 1mm, preferably at least 2mm, more preferably at least 3mm from the longitudinal axis.

When introducing the tip end of the head portion no deeper into the ear canal than to the border between the outer part and the inner part of the outer ear canal of the subject's outer ear, there is the risk that artifacts, such as earwax, hair and other kind of dirt from the outer part of the outer ear canal obstruct the view of the small electronic imaging unit onto the subject's eardrum. Therefore, it is advantageous to capture several images from different positions within the ear canal. For doing so, the otoscope adapted for performing the method according to the present invention may comprise more than one electronic imaging unit at the distal end of its head portion, e.g. two imaging units, located at different positions on the head portion.

In another preferred embodiment, the otoscope may further comprise a motion mechanism configured to allow displacement of the electronic imaging unit relative to the handle portion. With such a motion mechanism, it is possible to capture a plurality of images from different positions by one single electronic imaging unit within the subject's ear canal, thereby avoiding the need for two or more electronic imaging units.

The motion mechanism is preferably configured to allow for at least partial rotation of the electronic imaging unit about an axis of rotation. The axis of rotation may correspond to the longitudinal axis of the head portion. By displacing the electronic imaging unit along a predefined motion path, it is possible to automatically calculate the distance of the electronic imaging unit to the detected objects, as described above. In view of the typical size of objects found in the ear canal, such as hair and earwax particles, the motion mechanism preferably allows for displacement of the electronic imaging unit of at least 1mm within the subject's ear canal. A rotation of at least 90°, more preferably of at least 120°, even more preferably of 180° or even more degrees around the axis of rotation may be realized. Preferably, the motion mechanism allows for rotation in both directions, i.e. clockwise and counter-clockwise. The motion mechanism may also allow for rotational displacement about more than one axis. The motion mechanism may comprise at least one motor and one or more gears and/or bearings. The electronic imaging unit may be connected to a flexible cable, e.g. a flexible ribbon cable, to allow for such a movement.

Preferably, the electronic imaging unit is tilted against the axis of rotation so as to be continuously directed to a predetermined point on the axis of rotation, the predetermined point having a fixed distance to the electronic imaging unit. In view of the typical length of the inner part of the outer ear canal of the subject's outer ear, the distance may be between 3mm and 20mm. Thus, the optical axis ("viewing direction") of the electronic imaging unit is optimized for centering on the eardrum.

The head portion of the otoscope for carrying out the inventive method is preferably shaped in such a way that its distal end comprising the electronic imaging unit can be introduced only as deep into the ear canal as not to touch the eardrum. The ear canal of the subject's outer ear is limited by the eardrum. Notably, the ear canal of the subject's outer ear comprises an outer part which refers to a portion of the subject's outer ear (i.e. the subject's external auditory canal) that is surrounded by soft connective tissue and that usually contains hair and earwax. The outer part comprises approximately the outer half of the ear canal of the subject's outer ear. Furthermore, the ear canal of the subject's outer ear also comprises an inner part which refers to a portion of the subject's outer ear (i.e. the subject's external auditory canal) that is surrounded by hard skull bone and that is usually free from any hair and earwax. This portion extends from the proximal end of the outer part of the ear canal of the subject's outer ear to the eardrum. The inner part of the ear canal is very sensitive to pain in case of injury by mechanical friction. Injuring the inner part of the ear canal even bears the risk of cardiovascular complications through vagal overstimulation.

Preferably, a tip portion of the distal end can be introduced into the ear canal of the subject's outer ear no further than to a distance from the eardrum of at least a few millimeters, preferably of at least 3mm.

The tapering head portion of the otoscope for performing the method according to the present invention can be shaped with a blunt, rounded tip end, as compared to a conventionally known otoscope, thereby reducing the risk of introducing injury or discomfort to the subject. Thus, the device can be securely handled by laypersons. The otoscope adapted for performing the method according to the present invention, nevertheless, allows detecting the eardrum, since the electronic imaging unit is provided at the distal end of the head portion.

Preferably, the distal end of the head portion is provided with a round and smooth shape. Moreover, the distal end may be made from a relatively soft material, such as silicone, or it may comprise an outer surface made of such a soft material. Furthermore, the longitudinal force upon introduction into the ear canal can be limited by a telescoping mechanism or the use of an elastic element.

The functional concept of a otoscope of the art as described above, however, requires the tip end of the head portion to be relatively small and acute (sharp), usually having a diameter of only about 3mm. It is noted that the diameter of the inner part of the outer ear canal of an adult is about 4mm. Therefore, if the user (untrained) does not pay attention, the tip portion might be introduced deeply into the inner part of the outer ear canal causing serious injuries to the subject. To substantially avoid this risk, the head portion of the otoscope adapted for carrying out the method according to the present invention (also having a tapered shape) preferably exhibits a diameter of at least 4mm, preferably of more than 5mm, more preferably of more than 6mm, at a position along the longitudinal axis of the head portion of no more than 4mm from a distal end point of the head portion. Thus, it is geometrically excluded to introduce the distal end of the head portion too far into the subject's ear canal. Different geometries of tapers may preferably be used according to the age group of the subject. For children, for example, the head portion of the otoscope adapted to carry out the method according to the present invention may exhibit a diameter of about 5mm at a position along the longitudinal axis of the head portion of no more than 4mm away from a distal end point of the head portion.

For hygienic reasons, the otoscope adapted for carrying out the method according to the present invention preferably further comprises an at least partially transparent probe cover configured to be put over the head portion. The probe cover may be made from a plastic material, preferably from a transparent plastic material. Such a probe cover may be designed as a single-use product that can be produced in larger numbers with low costs. The probe cover shall be transparent, at least at the locations where it covers the electronic imaging unit, so as to allow the electronic imaging unit to have a clear view onto the eardrum. The probe cover also inhibits contamination of the head portion of the otoscope comprising the electronic imaging unit, in particular when introducing the head portion into the subject's ear canal.

Preferably, the probe cover is adapted to be fixed to at least one section of either the head portion and/or the handle portion in such a way that the probe cover does not move relative to the handle portion during displacement of the electronic imaging unit by the motion mechanism. Otherwise, artifacts, such as earwax particles, adhering to the probe cover will depicted by the electronic imaging unit, even if the electronic imaging unit is displaced by the motion mechanism. This, however, would interfere with object identification (e.g. if the object to be identified is the eardrum) and elimination of artifacts from the captured images.

The otoscope adapted for carrying out the method according to the present invention may further comprise a probe cover moving mechanism adapted to move at least a portion of the probe cover with respect to the electronic imaging unit. Thus, artifacts, such as earwax particles, adhering to the probe cover and obstructing the view of the electronic imaging unit onto the eardrum can be moved away from the electronic imaging unit by the probe cover moving mechanism.

Preferably, the probe cover is designed in a way that allows unfolding or peeling of portions of the probe cover in order to move portions of the probe cover contaminated e.g. with earwax away from the electronic imaging unit. The method according to the present invention may further comprise a step of moving the probe cover against the electronic imaging unit or vice versa.

To illuminate the subject's ear canal and eardrum, the otoscope adapted to carry out the inventive method may further comprise at least one light source typically positioned at the distal end of the head portion. The term "light source" is understood to apply to any source emitting photons.

Since geometrical restrictions limit the space at the distal end of the head portion, the light source is preferably formed by the distal end of a light guide. For example, the light guide may exhibit a diameter of less than 1mm, preferably of less than 0.5mm, more preferably of about 0.2mm. The light guide may be connected to an LED located remote from the distal end of the head portion. The light guide may be e.g. a nylon light guide, preferably having a diameter of only about 0.2mm to 1mm. Alternatively, a light source may be formed e.g. by a small light emitting diode that is placed directly at the distal end of the head portion.

It is advantageous, if the otoscope adapted to carry out the inventive method comprises a plurality of light sources at the distal end of the head portion, preferably with each light source being separately controllable. By illuminating objects in the subject's ear canal from different positions, e.g. by sequentially switching on and off the individual light sources, it may also be envisaged to distinguish different objects in the ear, without necessarily having to displace the electronic imaging unit by a motion mechanism within the ear canal. An object relatively far away from the electronic imaging unit, such as the eardrum, will change its appearance only slightly when being illuminated from different positions at the distal end of the head portion. However, artifacts that are relatively close to the electronic imaging unit (such as hair and earwax) will change their appearance (position) drastically. The otoscope therefore preferably comprises means, in particular a logic unit, such as a microprocessor, adapted to distinguish different objects in the subject's ear based on images taken with the objects being illuminated from different positions.

Additionally or alternatively, the at least one light source may be controlled in view of the color, so that the color of the light emitted by the light source is changed. For example green color may be preferred to recognize earwax.

Like the electronic imaging unit, the at least one light source is preferably positioned radially offset from the longitudinal axis of the head portion. Such a configuration allows illumination of the eardrum without the need to introduce the light source as deeply into the ear canal as it would be necessary, if the light source were placed centrally on the longitudinal axis of the head portion. The offset may be at least 1mm, preferably at least 2mm, more preferably at least 3mm from the longitudinal axis.

Preferably, the at least one light source is arranged so as to maintain a predetermined distance with respect to the electronic imaging unit, even when the electronic imaging unit is displaced by the motion mechanism. Such a configuration is advantageous, because the predetermined distal relationship between the at least one light source and the electronic imaging unit allows for improved (automatic) image analysis. If a motion mechanism is provided, the motion mechanism preferably also displaces the at least one light source. If the light source is provided in the form of a light guide, the light guide should be sufficiently flexible to allow for such a displacement of the at least one light source.

The otoscope adapted for carrying out the inventive method may further comprise a logic unit, such as a microprocessor. The logic unit may be adapted to control the electronic imaging unit and/or the at least one light source. The logic unit may analyze the images obtained by the electronic imaging unit e.g. in order to compare two images obtained with the electronic imaging unit located at different positions within the ear and/or with the object illuminated from different positions, so as to identify and discriminate different objects in the subject's ear. The logic unit may further be adapted to generate or calculate a new image wherein predetermined objects that have been previously identified are eliminated.

An exemplary embodiment of an otoscope adapted for carrying out the method of the present invention will be described in more detail in the following with respect to the drawings, wherein:
- figure 1: schematically shows a cross-sectional view of a head portion and of a part of a handle portion of an embodiment of an otoscope for carrying out the inventive method;
- figure 2: shows an enlarged view of a plate covering a bore provided in the head portion illustrated in figure 1; and
- figure 3: shows an otoscope of the prior art, with its head portion partially introduced into the subject's ear canal.

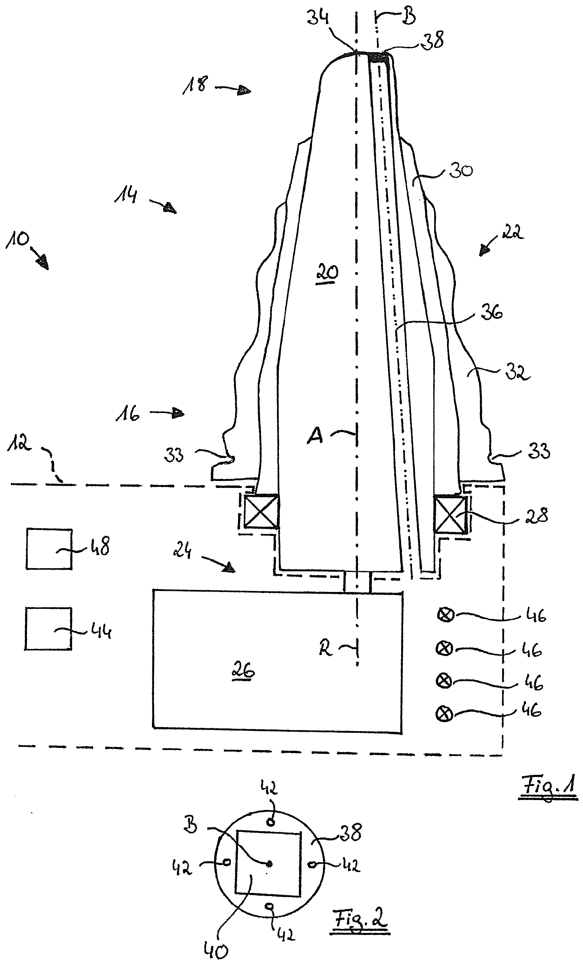

Figure 1 schematically shows a cross-sectional view of a head portion 14 and a part of a handle portion 12 (only shown in phantom lines) of an embodiment of an otoscope 10 adapted for carrying out the method according to the present invention. As can be seen from figure 1, the head portion 14 has a substantially tapering form extending along a longitudinal axis A of the head portion 14. The head portion 14 comprises a relatively large proximal end 16 adjacent to the handle portion 12 and a smaller distal end 18. The distal end 18 of the head portion 14 is adapted to be introduced into a subject's ear canal.

Furthermore, the head portion 14 comprises a rotatable, radial inner portion 20 and a fixed, radial exterior portion 22. The rotatable portion 20 is rotatable about an axis of rotation R which - in the shown exemplary embodiment - corresponds to the longitudinal axis A of the head portion 14. A motion mechanism 24 comprising a servo motor 26 is positioned within the handle portion 12 and is coupled to the rotatable portion 20 of the head portion 14, so as to rotate the rotatable portion 20 about its axis of rotation R relative to the fixed portion 22 of the head portion and relative to the handle portion 12 of the otoscope 10. The rotatable portion 20 is supported by a radial bearing 28 (also only schematically shown).

In the exemplary embodiment shown, the exterior portion 22 of the head portion 14 comprises a support structure 30 providing the required stability to the head portion 14. The support structure is at least partially covered by an outer cladding 32 formed from a relatively soft material, such as silicone. The cladding 32 makes it more comfortable for the subject to introduce the distal end 18 of the head portion 14 into his ear canal. The cladding 32 may comprise a circular slot-like recess 33 adapted to engage with a complementarily formed circular tongue of a probe cover (not shown). The probe cover may be formed from a plastic material and may be adapted to be put over the head portion 14. Preferably, the probe cover is formed from a transparent material. Its wall may be relatively thin, thereby making the probe cover relatively flexible. At least a portion of the probe cover covering the distal end 18 of the head portion 14 should be transparent, so as to allow an electronic imaging unit (described in the following) which is located at the distal end 18 of the head portion 14 to have a free view through the probe cover. For hygienic reasons, the probe cover is preferably designed as a single-use product. The probe cover also reliably inhibits contamination of the distal end 18 comprising the electronic imaging unit. Without such a probe cover there is a high risk that e.g. earwax particles may adhere to the electronic imaging unit (thereby deteriorating the image quality thereof) when introducing the distal end 18 into the outer part of the ear canal of the subject.

The head portion 14 comprises a distal end point 34 which, in the shown exemplary embodiment, is located substantially on the longitudinal axis A of the head portion 14. However, the head portion 14 might alternatively have a tapering shape that is not substantially symmetrical to its longitudinal axis A (as shown in figure 1) but is more adapted to the anatomy of the human ear canal.

Irrespective of the precise shape of the head portion 14, the head portion 14 is preferably dimensioned in such a way that it cannot be introduced into the inner part of the ear canal of the subject's outer ear. In the exemplary embodiment shown, the distal end 18 of the head portion 14 has a substantially round shape. Only a few millimeters (less than 4mm) from the distal end point 34 in the direction of the longitudinal axis A, the head portion 14 exhibits a diameter of more than 5mm. Since the inner part of the ear canal of an adult usually exhibits a diameter of 4mm, there is no risk that the distal end 18 of the head portion 14 is inadvertently introduced too deeply into the subject's ear canal. Therefore, injuries to the sensitive skin of the inner part of the ear canal and/or to the eardrum can be reliably avoided.

The movable portion 20 comprises a bore 36 extending substantially along the axial direction A of the head portion 14, but not exactly parallel thereto. The distal end of the bore 36 is located in proximity to the distal end point 34, but offset with its bore axis B by at least 2mm from the longitudinal axis A. Furthermore, the distal end of the bore 36 is closed by a plate 38. An enlarged top view of the plate 38 is shown in figure 2. Since the bore 36 is cylindrical in shape, the plate 38 has a generally circular appearance in figure 2 with the bore axis B forming the center thereof. However, the bore 30 and/or the plate 38 may equally exhibit other shapes.

The plate 38 supports a wide-angle color video camera 40 and distal ends of four light guides 42. In the exemplary embodiment, the light guides 42 are located around the video camera 40, such that one light guide 42 is associated with each of the four lateral sides of the substantially rectangular video camera 40. However, this is not a prerequisite for the present invention. Instead of four light guides 42, for example, only two light guides 42 may be provided in the otoscope 10. The video camera 40 is advantageously a wafer-level camera of dimensions between 1mm and 2mm having a substantially flat configuration. The wafer-level camera advantageously exhibits dimensions of only about 1 mm x 1 mm providing a resolution of about 250 pixels of 250 pixels. The plate 38 has a diameter between 1.5mm and 2.0mm and the light guides 42 have a diameter of only about 0.2mm.

The video camera 40 is connected to a distal end of a cable (not shown). The cable, e.g. a ribbon cable, extends through the bore 36 and into the handle portion 12 of the otoscope 10. A distal end of the cable is connected to a logic unit 44, such as a microprocessor, which is schematically illustrated in figure 1. Similarly, the light guides 42 (not shown in figure 1) extend through the bore 36 and into the handle portion 12 of the otoscope 10. Proximal ends of the light guides 42 are connected to four LEDs 46, respectively. The LEDs 46 are positioned - like the logic unit 44 - within the handle portion 12 of the otoscope 10. The LEDs 46 can be switched on and off individually. Furthermore, the handle portion 12 preferably comprises a memory 48 for storing images captured by the video camera 40. The memory may be formed e.g. by a storage card slot and a corresponding storage card inserted in the slot. The handle portion 12 may further comprise a display (not shown) for displaying the images taken by the camera 40 to the user. Additionally or alternatively, the handle portion 12 may comprise a cable connection port, such as a USB-port, and/or a wireless connection, such as Bluetooth® or WIFI®, and/or an energy supply, such as a (rechargeable) battery. These additional (optional) components of the handle portion 12 are known e.g. from digital cameras.

For capturing images of a subject's inner part of the ear canal, and in particular of a subject's eardrum, the distal end 18 of the head portion 14 has to be introduced into the subject's ear canal. Due to the shape of the head portion 14 there is no risk to insert the distal end 18 too deeply into the ear canal. That is, the shape and geometry of the distal end 18 does not allow for significantly introducing the distal end point 34 into the subject's inner part of the ear canal which is very pain-sensitive. Therefore, injuries to the skin of the inner part of the ear canal and/or the eardrum can be reliably avoided. The geometry and the technology of the inventive otoscope do not require deforming the subject's ear as with an otoscope of the art, as described above. Consequently, the otoscope adapted to carry out the method according to the present invention can also be securely applied by laypersons.

Even though the distal end 18 of the head portion 14 will not be inserted into the inner part of the ear canal, the otoscope, nevertheless, allows for capturing images from the inner part of the ear canal and the eardrum, because of the wide angle camera 40 being provided at the distal end 18 of the head portion 14. In order to improve the ability of the camera 40 to "see" the eardrum, the camera 40 is placed offset from the longitudinal axis A of the head portion 14. Furthermore, the main "viewing direction" of the camera 40, corresponding to the bore axis B, is angled with respect to the longitudinal axis A of the head portion 14. The bore axis B and the longitudinal axis A intersect at a point having a predetermined distance from the distal end point 34, wherein the predetermined distance corresponds to the typical length of a subject's inner part of the ear canal, so that the camera 40 is directed to the eardrum.

When the distal end 18 of the head portion is introduced in the subject's ear canal, it may happen that objects, such as earwax particles or hair, in front of the camera 40, e.g. adhering to the probe cover, partially or even fully obstruct the view onto to eardrum. Therefore, the motion mechanism 24 may turn the rotatable portion 20 of the head portion 14 with respect to the remaining otoscope 10 about its axis of rotation R. For example, the motion mechanism 24 may rotate the rotatable portion 20 from an initial position by about 120° in clockwise direction, then from the initial position by about 120 in counter-clockwise direction, and finally return to the initial position. The camera 40 may capture one or more images from each of these equally spaced three positions. The logic unit 44 may identify different objects in the subject's ear by comparing the images received from the camera 40. In particular, the logic unit 44 may discriminate the eardrum from other objects by determining their distance to the camera 40 according to the principle of stereoscopic viewing, as described in more detail above.

Additionally or alternatively (preferably additionally) to the identification process described above, more than one image may be taken from each of the three positions of the camera 40, with different LEDs 46 switched on and off for each captured image. Illumination of the eardrum and other objects from different positions also assists to discriminate these objects, as described in more detail above.

Finally, a new image may be generated (preferably by the logic unit 44) in which objects, such as hair and earwax, are eliminated so as to clearly show the eardrum. The degree of reddishness of the eardrum can then be easily determined. The user may be provided with corresponding information, assisting him to decide as to whether see the physician, or not. Also if the otoscope failed to detect the eardrum because of massive earwax in the subject's ear canal, corresponding information may be provided to the user. The user may then decide to visit a physician for having his ear canal cleaned.

Alternatively, the otoscope may provide pictures showing only objects other than the eardrum, e.g. showing only an object that has been unintentionally introduced into the ear canal, such as a pencil tip.

### Items

1. Method of identifying objects in a subject's ear, comprising the following steps:
   - introducing at least one electronic imaging unit (40) and at least one light source into an ear canal of a subject's outer ear;
   - using the at least one electronic imaging unit (40) to capture at least one image; and
   - determining brightness and/or color information to identify objects shown in the at least one image.
2. Method according to item 1, further comprising the following steps:
   - using the at least one electronic imaging unit (40) to capture at least two images from different positions within the ear canal and/or with illumination from different positions within the ear canal; and
   - comparing the at least two captured images with each other to identify objects shown in the images.
3. Method according to item 2, further comprising:
   - discriminating different objects, such as the eardrum and artifacts, by comparing their positions in at least two images captured from different positions within the ear canal.
4. Method according to item 2 or 3, further comprising:
   - discriminating different objects, such as earwax, hair, and the eardrum, by comparing their appearance in at least two images captured with illumination from different positions within the ear canal.
5. Method according to any one of items 2-4, wherein the method further comprises a step of:
   - generating a calculated image based on the at least two captured images, wherein the calculated image preferably does not exhibit objects, such as earwax and hair, located relatively close to the electronic imaging unit, once the objects within the subject's ear have been identified
6. Method according to any one of the preceding items, wherein the at least one electronic imaging unit (40) is a color video camera (40), preferably a wide angle color video camera (40), and wherein the method further comprises a step of:
   - determining the degree of reddishness of the eardrum, once the eardrum has been identified.
7. Method according to any one of the preceding items, wherein the method further comprises a step of:
   - informing the user correspondingly, if identification of the eardrum has failed.
8. Method according to any one of the preceding items, wherein the at least one electronic imaging unit (40) and/or the at least one light source is displaced within the ear canal of the subject's outer ear along a predetermined path and/or by a predetermined distance between the moment of capturing a first image and the moment of capturing a second image.
9. Method according to any one of the preceding items, wherein the at least one electronic imaging unit (40) and the at least one light source are introduced into the ear canal of the subject's outer ear no further than to a distance from the eardrum of at least a few millimeters, preferably of at least 3mm.
10. Method according to any one of the preceding items, wherein an otoscope (10) is used to carry out the method, the otoscope (10) comprising:
   - a handle portion (12) allowing a user to manipulate the otoscope (10) during its application; and
   - a head portion (14) exhibiting a substantially tapering form extending along a longitudinal axis (A) of the head portion (14), wherein the head portion (14) has a proximal end (16) adjacent to the handle portion (12) and a smaller distal end (18) configured to be introduced into the ear canal of the subject's outer ear,
   wherein the otoscope (10) further comprises the at least one electronic imaging unit (40) positioned in the distal end (18) of the head portion (14).
11. Method according to item 10, wherein the electronic imaging unit (40) is positioned radially offset from the longitudinal axis (A) of the head portion (14).
12. Method according to item 10 or 11, wherein the otoscope (10) further comprises a motion mechanism (24) configured to allow displacement of the electronic imaging unit (40) relative to the handle portion (12).
13. Method according to any one of items 10 to 12, wherein the motion mechanism (24) is configured to allow for at least partial rotation of the electronic imaging unit (40) about an axis of ration (R), wherein the axis of rotation (R) preferably corresponds to the longitudinal axis (A) of the head portion (14).
14. Method according to any one of items 10 to 13, wherein the electronic imaging unit (40) is tilted against the axis of rotation (R) so as to be continuously directed to a predetermined point on the axis of rotation (R), the predetermined point having a fixed distance to the electronic imaging unit (40).
15. Method according to any one of items 10 to 14, wherein the head portion (14) is shaped in such a way that its distal end (18) comprising the at least one electronic imaging unit (40) can be introduced only as deep into the ear canal as not to touch the eardrum.
16. Method according to any one of items 10 to 15, wherein the head portion (14) exhibits a diameter of more than 4mm, preferably of more than 5mm, more preferably of more than 6mm, at a position along the longitudinal axis (A) of no more than 4mm from a distal end point (34) of the head portion (14).

## Claims

1. Method of identifying objects in a subject's ear, comprising the following steps:
- introducing at least one electronic imaging unit (40) and at least one light source into an ear canal of a subject's outer ear;
- using the at least one electronic imaging unit (40) to capture at least one image; and
- determining brightness and/or color information to identify objects shown in the at least one image.

2. Method according to claim 1, further comprising the following steps:
- using the at least one electronic imaging unit (40) to capture at least two images from different positions within the ear canal and/or with illumination from different positions within the ear canal; and
- comparing the at least two captured images with each other to identify objects shown in the images.

3. Method according to claim 2, further comprising:
- discriminating different objects, such as the eardrum and artifacts, by comparing their positions in at least two images captured from different positions within the ear canal.

4. Method according to claim 2 or 3, further comprising:
- discriminating different objects, such as earwax, hair, and the eardrum, by comparing their appearance in at least two images captured with illumination from different positions within the ear canal.

5. Method according to any one of claims 2-4, wherein the method further comprises a step of:
- generating a calculated image based on the at least two captured images, wherein the calculated image preferably does not exhibit objects, such as earwax and hair, located relatively close to the electronic imaging unit, once the objects within the subject's ear have been identified

6. Method according to any one of the preceding claims, wherein the at least one electronic imaging unit (40) is a color video camera (40), preferably a wide angle color video camera (40), and wherein the method further comprises a step of:
- determining the degree of reddishness of the eardrum, once the eardrum has been identified.

7. Method according to any one of the preceding claims, wherein the method further comprises a step of:
- informing the user correspondingly, if identification of the eardrum has failed.

8. Method according to any one of the preceding claims, wherein the at least one electronic imaging unit (40) and/or the at least one light source is displaced within the ear canal of the subject's outer ear along a predetermined path and/or by a predetermined distance between the moment of capturing a first image and the moment of capturing a second image.

9. Method according to any one of the preceding claims, wherein the at least one electronic imaging unit (40) and the at least one light source are introduced into the ear canal of the subject's outer ear no further than to a distance from the eardrum of at least a few millimeters, preferably of at least 3mm.

10. Method according to any one of the preceding claims, wherein an otoscope (10) is used to carry out the method, the otoscope (10) comprising:
- a handle portion (12) allowing a user to manipulate the otoscope (10) during its application; and
- a head portion (14) exhibiting a substantially tapering form extending along a longitudinal axis (A) of the head portion (14), wherein the head portion (14) has a proximal end (16) adjacent to the handle portion (12) and a smaller distal end (18) configured to be introduced into the ear canal of the subject's outer ear,
wherein the otoscope (10) further comprises the at least one electronic imaging unit (40) positioned in the distal end (18) of the head portion (14).

11. Method according to claim 10, wherein the electronic imaging unit (40) is positioned radially offset from the longitudinal axis (A) of the head portion (14).

12. Method according to claim 10 or 11, wherein the otoscope (10) further comprises a motion mechanism (24) configured to allow displacement of the electronic imaging unit (40) relative to the handle portion (12).

13. Method according to any one of claims 10 to 12, wherein the motion mechanism (24) is configured to allow for at least partial rotation of the electronic imaging unit (40) about an axis of ration (R), wherein the axis of rotation (R) preferably corresponds to the longitudinal axis (A) of the head portion (14).

14. Method according to any one of claims 10 to 13, wherein the electronic imaging unit (40) is tilted against the axis of rotation (R) so as to be continuously directed to a predetermined point on the axis of rotation (R), the predetermined point having a fixed distance to the electronic imaging unit (40).

15. Method according to any one of claims 10 to 14, wherein the head portion (14) is shaped in such a way that its distal end (18) comprising the at least one electronic imaging unit (40) can be introduced only as deep into the ear canal as not to touch the eardrum.

16. Method according to any one of claims 10 to 15, wherein the head portion (14) exhibits a diameter of more than 4mm, preferably of more than 5mm, more preferably of more than 6mm, at a position along the longitudinal axis (A) of no more than 4mm from a distal end point (34) of the head portion (14).
